# FASCICULE DE BREVET EUROPEEN

(11) **EP 1 265 908 B1**
(45) Date de publication et mention de la délivrance du brevet: **03.11.2004**
(21) Numéro de dépôt: 01919556.9
(22) Date de dépôt: 23.03.2001
(51) Int. Cl.: C07H 17/08

(54) **PROCEDE POUR LA PREPARATION DE DERIVES 9-DEOXO-8A-AZA-(8A-ALKYL)-8A-HOMOERYTHROMYCINE A A PARTIR DE LA 9-DEOXO-9(Z)-HYDROXYIMINOERYTHROMYCINE A**
VERFAHREN ZUR HERSTELLUNG VON 9-DEOXO- 8A-AZA-(8A-ALKYL)-HOMOERYTHROMYCINE A DERIVATE VON 9-DEOXO-9(Z) - HYDROXYIMINOERYTHROMYCINE A
METHOD FOR PREPARING 9-DEOXO-8A-AZA-(8A-ALKYL)-8A-HOMOERYTHROMYCIN A DERIVATIVES FROM 9-DEOXO-9(Z)-HYDROXYIMINOERYTHROMYCIN A

(30) Priorité: 24.03.2000 FR 0003807
(43) Date de publication de la demande: 18.12.2002
(73) Titulaire: MERIAL, 69002 Lyon (FR)
(72) Inventeur: LEON, Patrick, (FR); LHERMITTE, Frédéric, F-69360 Saint Symphorien D'Ozon (FR); ODDON, Gilles, F-69740 Genas (FR); PAUZE, Denis, F-69360 Solaize (FR)
(74) Mandataire: Nargolwalla, Cyra
(86) Numéro de dépôt international: PCT/FR2001/000897
(87) Numéro de publication internationale: WO 2001/072763

(56) Documents cités:
- EP-A- 0 508 726
- EP-A- 0 879 823
- WO-A-00/58327

## Description

La présente invention a pour objet un procédé pour la préparation de la 9-déoxo-8a-aza-8a-homoérythromycine A et de ses dérivés 8a-alkylé, à partir de la 9-déoxo-9(Z)-hydroxyiminoérythromycine A.

La présente invention concerne plus particulièrement le domaine des antibiotiques macrolides de type érythromycine et plus particulièrement leurs dérivés aza-macrolides qui font l'objet du brevet EP 508 699 et répondent à la formule générale suivante : 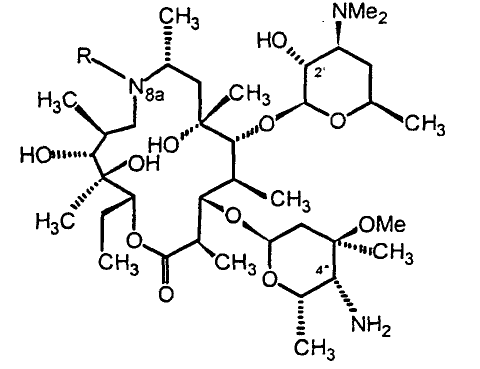 dans laquelle R représente un atome d'hydrogène, un groupement alkyle en C₁-C₁₀, alkényle en C₂-C₁₀ ou arylesulfonyle en C₆-C₁₂, le cas échéant substitués.

Ces composés sont obtenus à partir de l'érythromycine A et leur synthèse implique deux étapes majeures :
- la création du macrocycle 8a-azalide au départ de l'oxime 9-(E) de l'érythromycine A isomérisée en oxime 9-(Z) qui subit un réarrangement stéréospécifique de Beckmann et
- la modification du groupe cladinose en position 4" consistant en la transformation du 4"(S)-OH en 4"(R)-NH₂.

La présente invention concerne plus particulièrement la première étape et vise à proposer un nouveau procédé permettant de préparer la 9-déoxo-8a-aza-8a-homoérythromycine A, éventuellement 8a-alkylée, directement à partir de la 9-(Z)-oxime-érythromycine A de formule Il donnée ci-après.

La voie classique de synthèse peut être schématisée de la manière suivante :

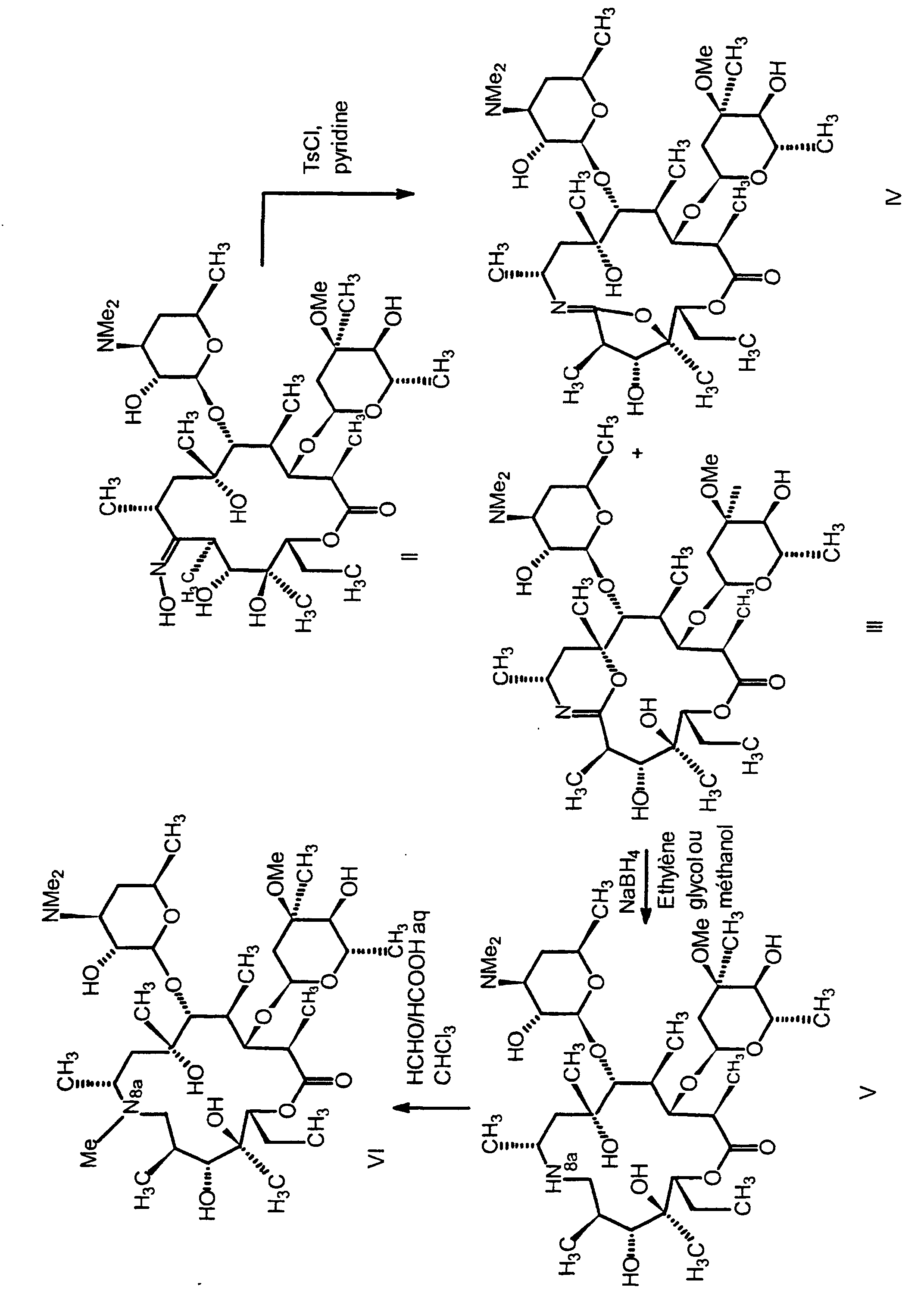

En fait, les conditions réactionnelles actuelles ne sont pas extrapolables à l'échelle industrielle.

Tout d'abord, elles nécessitent d'isoler les intermédiaires imidates III et IV. En effet, l'enchaînement de la réduction au borohydrure de sodium, directement sur le milieu réactionnel contenant lesdits intermédiaires imidates est inopérant du fait du caractère inhibiteur de la pyridine. De plus, ces intermédiaires sont peu stables et se déshydratent facilement à l'isolement. Il en résulte donc une chute significative du rendement.

D'autre part, l'imidate de formule IV est sensible à l'épimérisation en position 10, conduisant ainsi à une perte supplémentaire de produit.

Enfin, les conditions de réduction actuellement retenues à savoir borohydrure de sodium dans l'éthylène glycol ou le méthanol ne sont pas suffisamment efficaces à l'égard de l'imidate de formule III.

En conséquence, il est difficile dans les conditions expérimentales actuelles d'obtenir un rendement global de ces deux étapes à savoir réarrangement de Beckmann et réduction au borohydrure de sodium via l'isolement intermédiaire des deux imidates, qui soit supérieur à 30 %.

La présente invention a précisément pour objet de proposer une alternative efficace à la voie de synthèse discutée ci-dessus.

Plus précisément, la présente invention a pour objet un procédé de préparation d'un composé de formule générale V 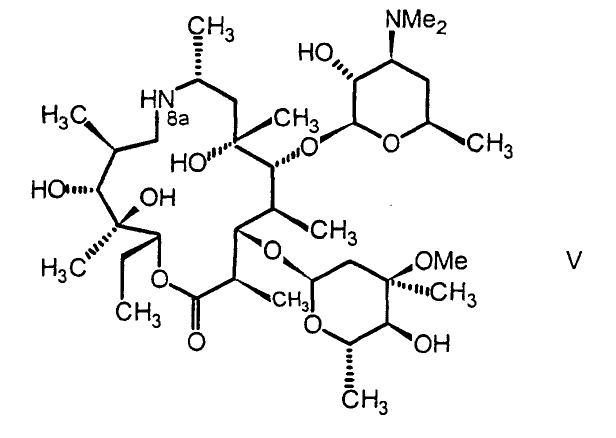 via le réarrangement stéréospécifique de Beckmann dans un milieu réactionnel utilisant la pyridine comme solvant principal, d'un composé de formule générale II 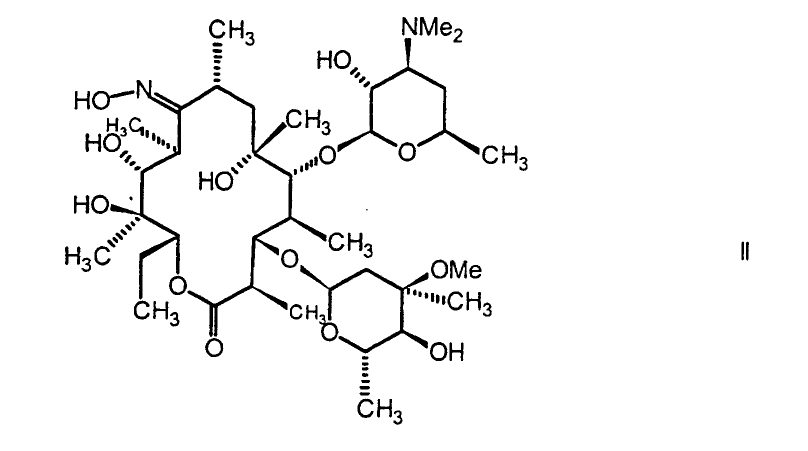 en deux intermédiaires imidates III et IV 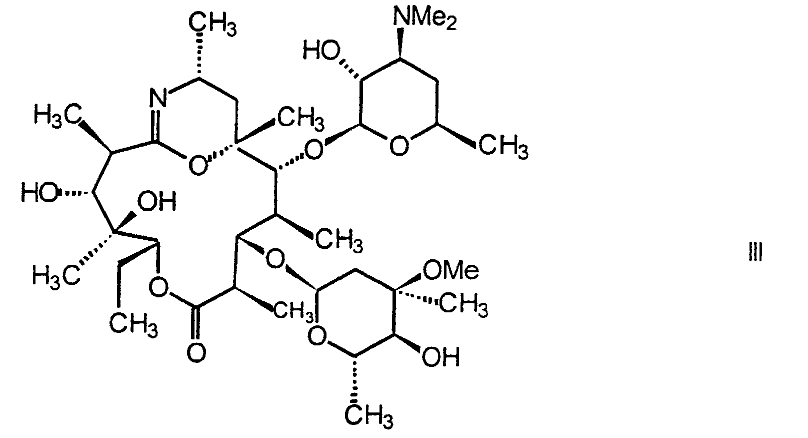 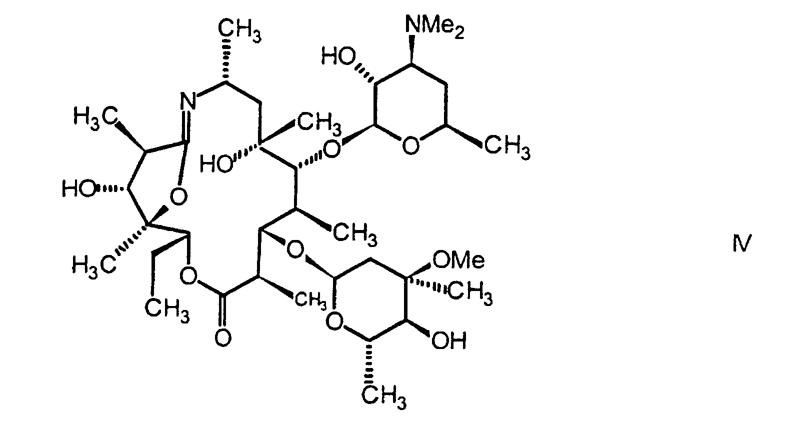 puis la réduction desdits composés III et IV caractérisé en ce que lesdits composés III et IV formés dans le milieu réactionnel du réarrangement de Beckmann ne sont pas isolés dudit milieu et sont directement engagés dans l'étape de réduction à l'aide d'une quantité suffisante de borohydrure, après extraction de la pyridine par un hydrocarbure miscible à celle-ci et dans lequel lesdits imidates III et IV, sous forme de sel, sont insolubles.

Selon une autre caractéristique, le procédé selon l'invention est en outre caractérisé par le fait que le composé de formule V formé à l'issu de la réaction de réduction n'est pas isolé du milieu réactionnel et y est directement N-alkylé par l'addition d'un réactif d'alkylation en quantité suffisante dans ledit milieu pour conduire à la 9-déoxo-8a-aza-8a-alkyl-8a-homoérythromycine A de formule VI.

De manière inattendue, les inventeurs ont constaté qu'il était possible de réaliser avec un rendement très satisfaisant, la réduction des composés intermédiaires imidates III et IV sans procéder à leur isolement préalable dudit milieu de réarrangement de Beckmann, sous réserve d'extraire la pyridine de ce milieu réactionnel à l'aide d'un hydrocarbure miscible à la pyridine et dans lequel les imidates III et IV, protonés au niveau du groupe 3'-diméthylamino, sont insolubles.

Il s'agit ainsi d'une démarche inverse à la voie classique qui consistait à extraire les imidates du milieu réactionnel, par le dichlorométhane après partage du résidu issu du réarrangement les contenant dans un mélange eau/dichlorométhane.

Un exemple de cette "voie classique" est décrit notamment dans EP-A-0 508 726.

De manière surprenante, l'utilisation d'un hydrocarbure permet d'extraire et d'éliminer la pyridine du milieu sans dégradation majeure de l'imidate IV et permet ainsi d'une part, de s'affranchir efficacement de l'étape d'isolement des intermédiaires imidates, qui est préjudiciable en termes de rendement et de faisabilité à l'échelle industrielle et d'autre part, de réaliser plus efficacement leur réduction par un borohydrure alcalin dans l'eau et/ou un solvant organique. Ceci a pour avantage de conduire à un rendement global nettement amélioré au regard des 30 % évoqués précédemment.

Par ailleurs, les inventeurs ont également mis en évidence que l'on pouvait effectuer la N-alkylation du composé réduit obtenu, directement dans le milieu réactionnel par addition d'un aldéhyde, sans qu'il soit nécessaire de l'isoler.

Ceci a pour avantages, notamment dans le cas de la N-méthylation, d'éviter la mise en oeuvre de chloroforme et d'acide formique comme dans le procédé Eschweiler-Clarke utilisé en particulier dans la demande EP 508 699 précitée, et de réaliser la réaction dans des conditions simplifiées du point de vue de la température et de la durée.

Le procédé selon l'invention va être décrit plus en détails ci-après en référence aux schémas réactionnels suivants :

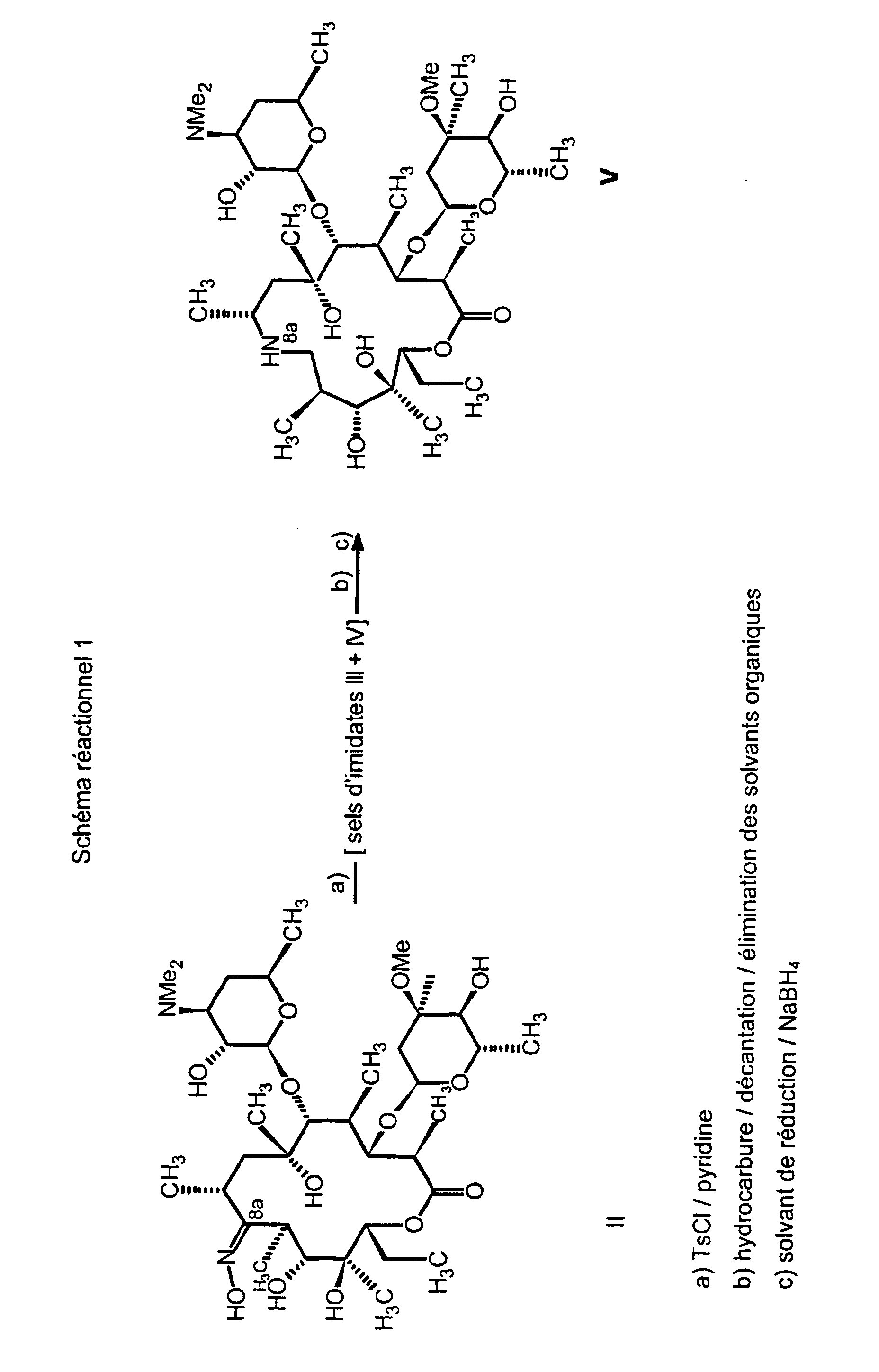

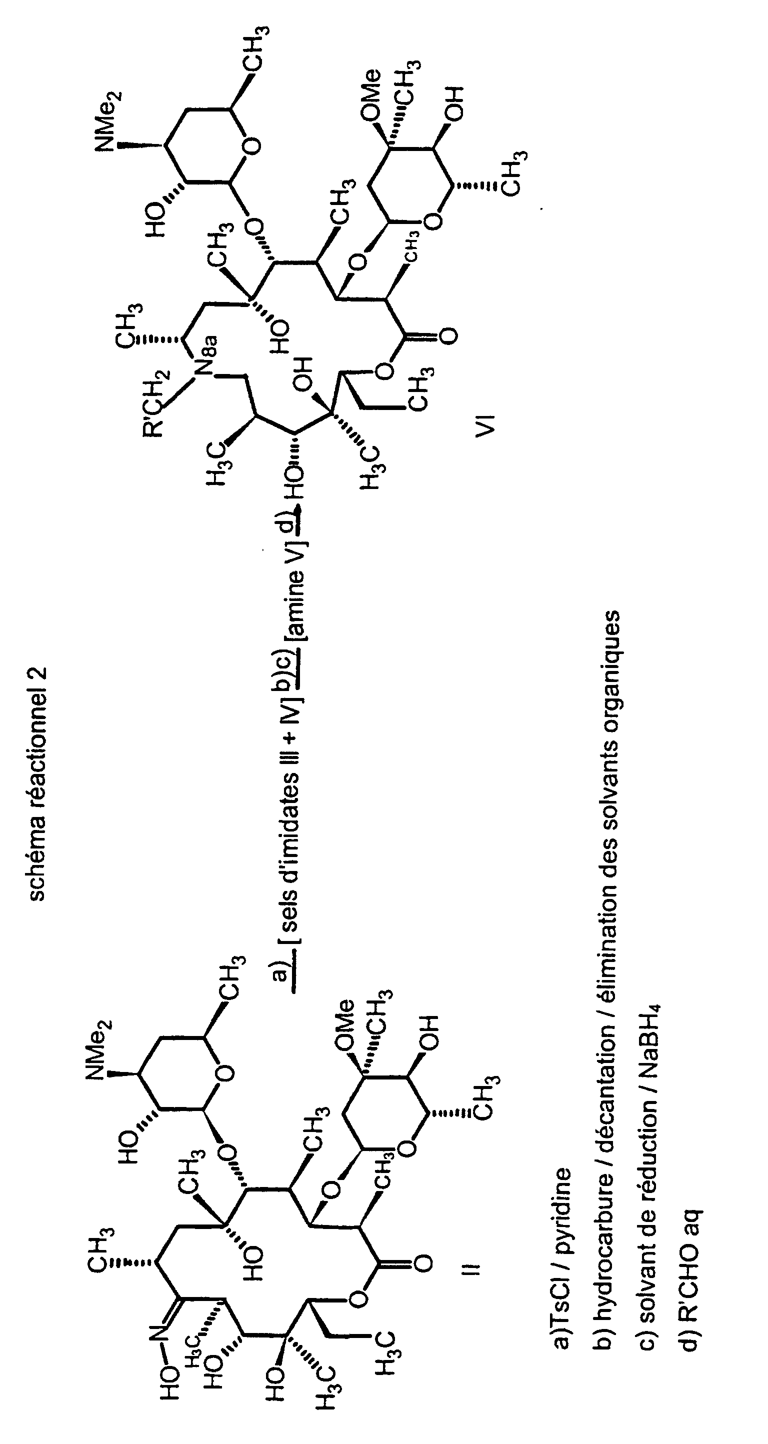

En référence au schéma réactionnel 1, la 9-(Z)-oxime de formule Il est tout d'abord soumise à un réarrangement de Beckmann dans un milieu à base de pyridine, conduisant à la formation des intermédiaires imidates III et IV qui ne sont pas isolés.

Ce réarrangement est effectué à l'aide de chlorure de sulfonyle choisi de préférence parmi le chlorure de tosyle, le chlorure de benzènesulfonyle et le chlorure de mésyle.

Selon un mode de réalisation préféré de l'invention, la 9-(Z)-oxime de formule Il est traitée dans la pyridine anhydre, par du chlorure de tosyle sous forme solide ou en solution dans un solvant organique, par exemple dans le toluène. La quantité de chlorure de tosyle est généralement comprise entre 1 et 10 équivalents par rapport à la 9-(Z)-oxime, de préférence entre 1,5 et 4 équivalents. Le solvant organique est généralement présent en quantité suffisante pour dissoudre le chlorure de tosyle. La réaction est réalisée à une température de préférence comprise entre 0 et 5°C.

Après réaction, on ajoute au milieu un hydrocarbure qui est miscible avec la pyridine et les autres solvants organiques présents dans ledit milieu réactionnel et dans lequel les intermédiaires imidates, protonés au niveau du groupe diméthylamino en position-3', ne sont pas solubles. L'hydrocarbure est choisi de préférence parmi les hydrocarbures à chaîne linéaire, ramifiée ou cyclique, contenant 5 à 15 atomes de carbone. A titre d'exemples représentatifs, on peut citer le pentane, le cyclohexane, le méthylcyclohexane et l'heptane. Il consiste plus préférentiellement en de l'heptane.

On laisse ensuite décanter le milieu et on élimine la phase supérieure comprenant les solvants organiques dont la pyridine.

On ajoute alors au résidu le solvant pour la réaction de réduction qui peut consister en de l'eau et/ou en un solvant organique choisi de préférence parmi les alcools en C₁-C₁₀, de préférence le méthanol ou l'isopropanol, les solvants du type amide, de préférence le N,N-diméthylformamide ou le N,N-diméthylacétamide, ou encore les solvants de type urée cyclique, de préférence la 1,3-diméthyl-3,4,5,6-tétrahydro-2(1H)-pyrimidone (DMPU) ou la 1,3-diméthyl-2-imidazolinone (DMEU).

On ajoute ensuite un borohydrure, de préférence le borohydrure de sodium ou de potassium, la température réactionnelle étant préférentiellement maintenue entre 0 et 5°C. La quantité de borohydrure est généralement comprise entre 3 et 15 équivalents par rapport au composé à réduire, de préférence entre 4 et 5 équivalents.

Après réaction, l'amine de formule V est soit isolée selon le schéma réactionnel 1, soit directement N-alkylée en position-8a selon le schéma réactionnel 2.

L'amine V peut ainsi être isolée du milieu réactionnel selon un mode opératoire conventionnel qui implique généralement des opérations d'extraction, de lavage puis de séchage.

Selon un autre mode de réalisation de l'invention, on peut transformer l'amine de formule V directement dans le milieu réactionnel de réduction en y ajoutant une quantité suffisante d'une aldéhyde de formule R'CHO avec R' représentant un atome d'hydrogène, un groupement alkyle en C₁-C₉, ou alkényle en C₂-C₉, de manière à obtenir un composé de formule générale VII 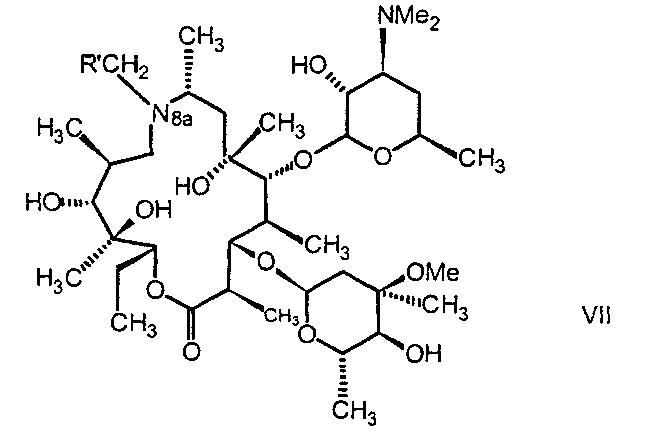 avec R' tel que défini ci-dessus.

L'aldéhyde utilisé est de préférence un aldéhyde en C₁ à C₄ et plus préférentiellement le formol, l'éthanal ou le propanal.

Dans une variante particulière de ce mode de réalisation du procédé revendiqué on ajoute du formol directement au milieu réactionnel de réduction contenant l'amine V, sans l'isoler. La température réactionnelle est comprise entre -10 et +30°C. La durée de réaction est en général de 2 heures.

L'amine 8a-N-méthylée de formule VI ainsi formée 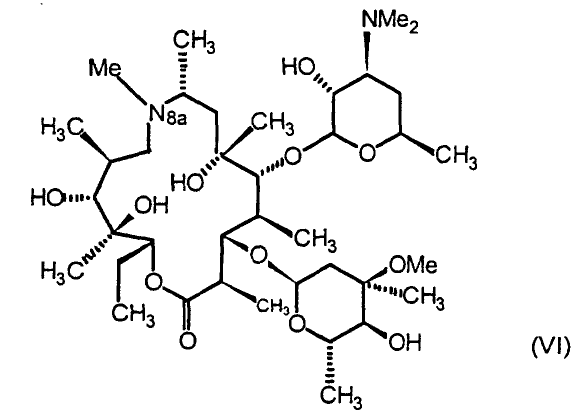 peut être isolée selon un mode opératoire conventionnel qui implique généralement des opérations d'extraction, de lavage puis de séchage.

Le procédé selon l'invention a donc pour avantage de permettre l'enchaînement des réactions de réarrangement de Beckmann sur la 9-(Z)-oxime-érythromycine A réalisé dans un milieu à base de pyridine et de la réduction des intermédiaires imidates ainsi formés par un borohydrure, sans recourir à leur isolement.

Selon un autre mode de réalisation, le procédé selon l'invention permet d'enchaîner une réaction d'alkylation telle une méthylation du composé réduit, directement dans le milieu réactionnel de réduction, dans des conditions simplifiées.

Les exemples figurant ci-après sont donnés à titre illustratif et non limitatif de la présente invention.

L'ensemble des essais est réalisé sous atmosphère inerte.

### EXEMPLE 1 :

### Préparation de la 9-déoxo-8a-aza-8a-homoérythromycine A (V) par addition de chlorure de tosyle solide et réduction dans l'eau, selon le schéma réactionnel 1 :

Dans un ballon sec et inerté à l'argon, on introduit 2 g de (9Z)-9-déoxo-9-hydroxyiminoérythromycine A (2,67 mmol) puis 16 ml de pyridine. La solution est refroidie à 0°C, puis on introduit par petites fractions 1,32 g de chlorure de tosyle (6,9 mmol, 2,6 éq.). Le milieu réactionnel est agité à 0 °C durant 1h30, puis on additionne à cette température 20 ml d'heptane. L'agitation est stoppée, le milieu réactionnel est alors laissé décanter. La phase supérieure est retirée par aspiration, 20 ml d'heptane sont de nouveau ajoutés sans agitation, puis la phase supérieure est retirée par aspiration.

Sous agitation et toujours à 0 °C, 10 ml d'eau distillée sont additionnés, puis 0,4g de borohydrure de sodium (10,5 mmol, 4 éq.) par petites fractions. Le milieu réactionnel est laissé remonter à température ambiante et agité une heure à cette température. 10 ml de méthanol sont alors ajoutés et après ½ heure, le milieu réactionnel est acidifié à l'aide d'une solution aqueuse 2N d'acide chlorhydrique jusqu'à pH = 3. La phase aqueuse est extraite par deux fois 10 ml de dichloromethane, les phases organiques sont éliminées, puis la phase aqueuse est basifiée à l'aide d'une solution aqueuse 2N de soude jusqu'à pH = 11. Après deux extractions par 20 ml de dichlorométhane, les phases organiques sont réunies, séchées sur sulfate de magnésium et concentrées sous vide.

On obtient 1,73 g d'un solide blanc d'une pureté dosée par HPLC de 69% en 9-déoxo-8a-aza-8a-homoérythromycine A, soit un rendement dosé de 60%.

### EXEMPLE 2 :

### Préparation de 9-déoxo-8a-aza-8a-homoérythromycine A (V) par addition d'une solution de chlorure de tosyle dans du toluène et réduction dans un mélange eau / isopropanol, selon le schéma réactionnel 1 :

Dans un ballon sec et inerté à l'argon, on introduit 10 g de (9Z)-9-déoxo-9-hydroxyiminoérythromycine A (13,35 mmol) puis 70 ml de pyridine. La solution est refroidie à 0°C, puis on introduit 6.6 g de chlorure de tosyle (34,5 mmol, 2,6 éq.) en solution dans 40 ml de toluène. Le milieu réactionnel est agité à 0 °C durant 1h30, puis on additionne à cette température 80 ml d'heptane. L'agitation est stoppée, le milieu réactionnel est alors laissé décanter. La phase supérieure est retirée par aspiration.

Sous agitation et toujours à 0 °C, 70 ml d'eau distillée et 30 ml d'isopropanol sont additionnés, puis 2 g de borohydrure de sodium (53 mmol, 4 éq.) par petites fractions. Le milieu réactionnel est laissé remonter à température ambiante et agité une heure à cette température. 10 ml de méthanol sont alors ajoutés et après ½ heure, le milieu réactionnel est acidifié à l'aide d'une solution aqueuse 2N d'acide chlorhydrique jusqu'à pH = 3. La phase aqueuse est extraite par deux fois 40 ml de dichlorométhane, les phases organiques sont éliminées, puis la phase aqueuse est basifiée à l'aide d'une solution aqueuse 2N de soude jusqu'à pH = 11. Après deux extractions par 40 ml de dichlorométhane, les phases organiques sont réunies, séchées sur sulfate de magnésium puis concentrées sous vide.

On obtient 8,6 g d'un solide blanc d'une pureté dosée par HPLC de 75,5% en 9-déoxo-8a-aza-8a-homoérythromycine A soit un rendement dosé de 65%.

### EXEMPLE 3 :

### Préparation de 9-déoxo-8a-aza-8a-homoérythromycine A (V) par addition du chlorure de tosyle en solution dans le toluène et réduction dans la N,N-diméthylformamide, selon le schéma réactionnel 1 :

Dans un ballon sec et inerté à l'argon, on introduit 20 g de (9Z)-9-déoxo-9-hydroxyiminoérythromycine A (26,7 mmol) puis 160 ml de pyridine. La solution est refroidie à 0°C, puis on introduit 10,5 g de chlorure de tosyle (53,4 mmol, 2 éq.) en solution dans 60 ml de toluène. Le milieu réactionnel est agité à 0 °C durant 1 h30, puis on additionne à cette température 260 ml d'heptane. L'agitation est stoppée, le milieu réactionnel est alors laissé décanter. La phase supérieure est retirée par aspiration.

Sous agitation et toujours à 0 °C, 200 ml de DMF sont additionnés, puis 4 g de borohydrure de sodium (106 mmol, 4 éq.) par petites fractions. Le milieu réactionnel est laissé remonter à température ambiante et agité une heure à cette température. 20 ml de méthanol sont alors ajoutés et après ½ heure, le milieu réactionnel est acidifié à l'aide d'une solution aqueuse 2N d'acide chlorhydrique jusqu'à pH = 3. La phase aqueuse est extraite par deux fois 50 ml de dichlorométhane, les phases organiques sont éliminées, puis la phase aqueuse est basifiée à l'aide d'une solution aqueuse 2N de soude jusqu'à pH = 11. Après deux extractions par 100 ml de dichlorométhane, les phases organiques sont réunies, séchées sur sulfate de magnésium puis concentrées sous vide.

On obtient 18,25 g d'un solide blanc d'une pureté dosée par HPLC de 64,6% en 9-déoxo-8a-aza-8a-homoérythromycine A soit un rendement dosé de 59%.

### EXEMPLE 4 :

### Préparation de la 9-déoxo-8a-aza-8a-méthyl-8a-homoérythromycine A (VI) par addition de chlorure de tosyle en solution dans du toluène et réduction dans un mélange eau / isopropanol, selon le schéma réactionnel 2 :

Dans un ballon sec et inerté à l'argon, on introduit 20 g de (9Z)-9-déoxo-9-hydroxyiminoérythromycine A (26,7 mmol) puis 160 ml de pyridine. La solution est refroidie à 0°C, puis on introduit 10.5 g de chlorure de tosyle (53,4 mmol, 2 éq.) en solution dans 60 ml de toluène. Le milieu réactionnel est agité à 0 °C durant 1h30, puis on additionne à cette température 240 ml d'heptane. L'agitation est stoppée, le milieu réactionnel est alors laissé décanter. La phase supérieure est retirée par aspiration.

Sous agitation et toujours à 0 °C, 140 ml d'eau et 60 ml d'isopropanol sont additionnés, puis 5 g de borohydrure de sodium (132.5 mmol, 5 éq.) par petites fractions. Le milieu réactionnel est laissé remonter à température ambiante et agité une heure à cette température.

22,8 g de formol aqueux 35 % (267 mmol, 10 éq.) sont ensuite ajoutés et le milieu réactionnel est abandonné 1 h30 à température ambiante. 20 ml de méthanol sont alors ajoutés et après ½ heure, le milieu réactionnel est acidifié à l'aide d'une solution aqueuse 2N d'acide chlorhydrique jusqu'à pH = 3. La phase aqueuse est extraite par deux fois 50 ml de dichlorométhane, les phases organiques sont éliminées, puis la phase aqueuse est basifiée à l'aide d'une solution aqueuse 2N de soude jusqu'à pH = 11. Après deux extractions par 100 ml d'acétate d'éthyle, les phases organiques sont réunies, séchées sur sulfate de magnésium puis concentrées sous vide.

On obtient 18,4 g d'un solide blanc d'une pureté dosée par HPLC de 69% en 9-déoxo-8a-aza-8a-méthyl-8a-homoérythromycine A soit un rendement dosé de 68%.

### EXEMPLE 5 :

### Préparation de la 9-déoxo-8a-aza-8a-méthyl-8a-homoérythromycine A (VI) par addition d'une solution de chlorure de tosyle dans du toluène et réduction dans un mélange eau / 1,3-diméthyl-2-imidazolinone (DMEU), selon le schéma réactionnel 2 :

Dans un ballon sec et inerté à l'argon, on introduit de la (9Z)-9-deoxo-9-hydroxyiminoerythromycine A (150 g, 0,19 mol; pureté 94 % p/p) puis de la pyridine (1089 g). La solution est refroidie à -10°C, puis on coule en 30 minutes du chlorure de tosyle (76,7 g, 0,4 mol, 2,1 équiv.) en solution dans du toluène (281 g). Le milieu réactionnel est ensuite agité entre -8 et -4°C durant 1 h30, puis on additionne à cette température de l'heptane (1011 g). L'agitation est stoppée, le milieu réactionnel est alors laissé à décanter. La phase supérieure est retirée par aspiration. La phase visqueuse inférieure est diluée une seconde fois sans agitation par de l'heptane (393 g). La phase supérieure est ensuite retirée par aspiration. Le milieu réactionnel est alors dilué avec de la DMEU (140,4 g) puis cette solution est additionnée à une solution de borohydrure de sodium (49,1 g, 1,28 mol, 6,85 équiv.) dans l'eau (1227 g). Après retour à température ambiante, le milieu réactionnel est agité 2 heures à cette température. Le milieu est ensuite traité par du méthanol (351,1 g) puis par du formol aqueux à 37% (140 g, 1,73 mol, 9,2 équiv.). On maintient à température ambiante pendant 2 h. Le milieu réactionnel est ensuite acidifié à l'aide d'une solution aqueuse 36% d'acide chlorhydrique jusqu'à pH = 4. La phase aqueuse est extraite par du toluène (355 g). La phase aqueuse est ensuite basifiée à l'aide d'une solution aqueuse de soude à 30% p/p jusqu'à pH = 10. Après deux extractions par de toluène (2x355 g) effectuées à 50°C, les phases organiques sont réunies, lavées à 50°C avec de l'eau (322 g) et concentrées sous vide. La phase organique ainsi obtenue est reprise plusieurs fois par de l'heptane pour distiller sous vide l'azéotrope heptane/pyridine.

On obtient 151 g d'un solide blanc d'une pureté dosée par HPLC de 65% en 9-deoxo-8a-aza-8a-méthyl-8a-homoerythromycine A soit un rendement dosé de 60%.

## Revendications

1. Procédé de préparation de la 9-déoxo-8a-aza-8a-homoérythromycine A de formule V 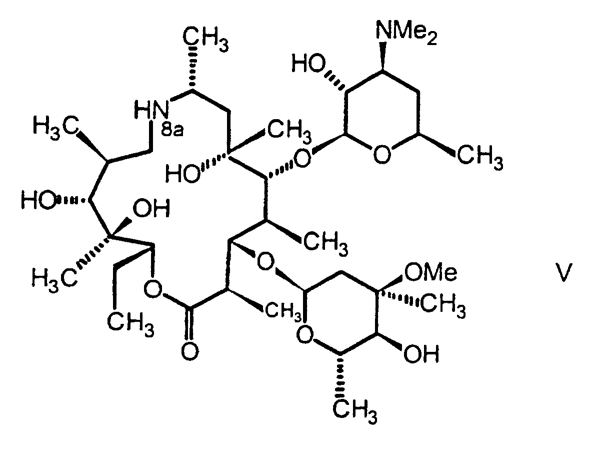 via le réarrangement stéréospécifique de Beckmann dans un milieu réactionnel utilisant la pyridine comme solvant principal, d'un composé de formule Il 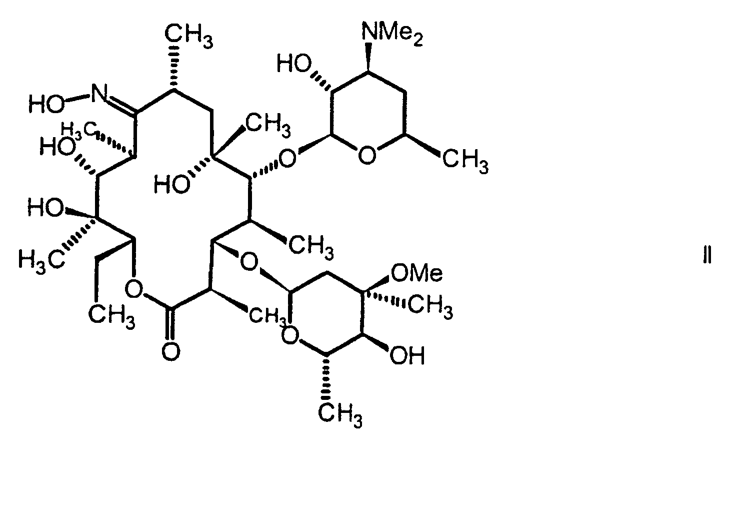 en deux intermédiaires imidates III et IV 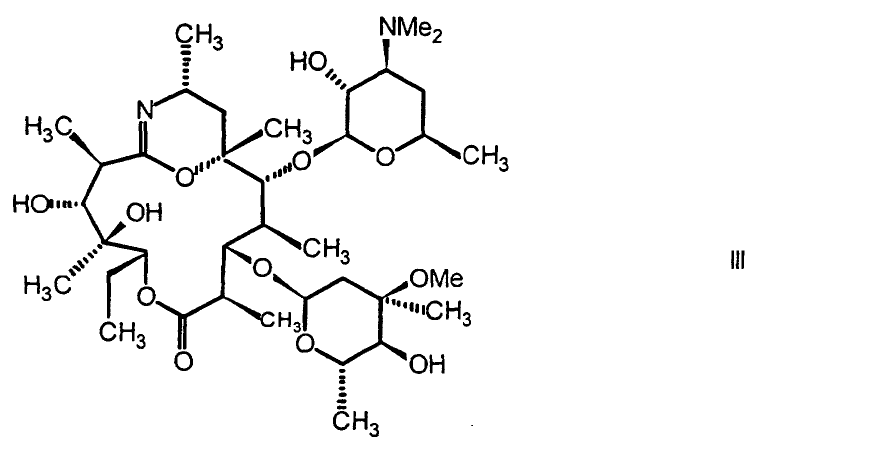 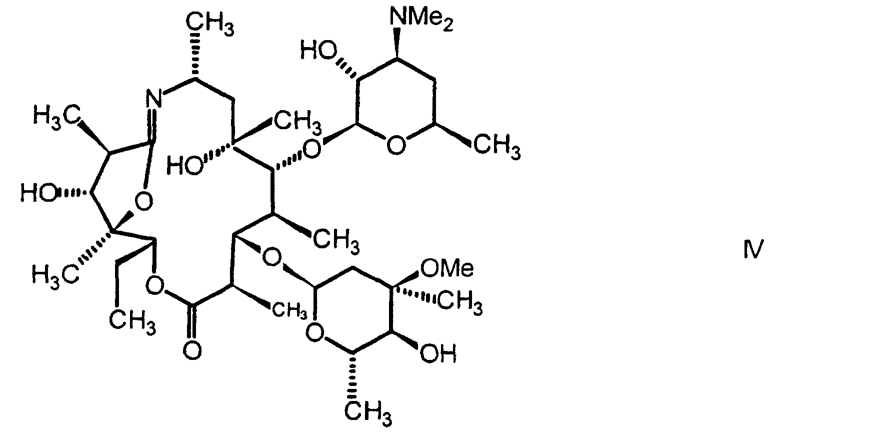 puis la réduction desdits composés III et IV **caractérisé en ce que** lesdits composés III et IV formés dans le milieu réactionnel du réarrangement de Beckmann ne sont pas isolés dudit milieu et sont directement engagés dans l'étape de réduction à l'aide d'une quantité suffisante d'un borohydrure, après extraction de la pyridine par un hydrocarbure miscible à celle-ci et dans lequel lesdits imidates III et IV sous forme de sel, sont insolubles.

2. Procédé selon la revendication 1, **caractérisé en ce que** le réarrangement de Beckmann est réalisé à l'aide de chlorure de sulfonyle, de préférence choisi parmi le chlorure de tosyle, le chlorure de benzènesulfonyle et le chlorure de mésyle.

3. Procédé selon la revendication 1 ou 2, **caractérisé en ce que** l'hydrocarbure est choisi parmi les hydrocarbures à chaîne linéaire, ramifiée ou cyclique, contenant 5 à 15 atomes de carbone, notamment parmi le pentane, le cyclohexane, le méthylcyclohexane et l'heptane, et consiste de préférence en de l'heptane.

4. Procédé selon l'une quelconque des revendications précédentes, **caractérisé en ce que** les solvants organiques présents dans le milieu pour le réarrangement de Beckmann, comprenant la pyridine, sont éliminés par décantation.

5. Procédé selon l'une quelconque des revendications 1 à 4, **caractérisé en ce que**, après l'élimination des solvants organiques dont la pyridine, du milieu du réarrangement de Beckmann, on ajoute au résidu le solvant de la réaction de réduction qui est de l'eau.

6. Procédé selon l'une quelconque des revendications 1 à 4, **caractérisé en ce que** après l'élimination des solvants organiques dont la pyridine, du milieu du réarrangement de Beckmann, on ajoute au résidu le solvant de la réaction de réduction qui est un solvant organique choisi parmi les alcools en C₁-C₁₀, de préférence le méthanol ou l'isopropanol, ou qui est un mélange d'un solvant précédent et d'eau.

7. Procédé selon l'une quelconque des revendications 1 à 4, **caractérisé en ce que** après l'élimination des solvants organiques dont la pyridine, du milieu du réarrangement de Beckmann, on ajoute au résidu le solvant de la réaction de réduction qui est de type amide et qui consiste de préférence en le N,N-diméthylformamide ou le N,N-diméthylacétamide, ou qui est un mélange d'un solvant précédent et d'eau.

8. Procédé selon l'une quelconque des revendications 1 à 4, **caractérisé en ce que** après l'élimination des solvants organiques dont la pyridine, du milieu du réarrangement de Beckmann, on ajoute au résidu le solvant de la réaction de réduction, qui est de type urée cyclique, de préférence la 1,3-diméthyl-3,4,5,6-tétrahydro-2(1H)-pyrimidone (DMPU) ou la 1,3-diméthyl-2-imidazolinone (DMEU), ou qui est un mélange d'un solvant précédent et d'eau.

9. Procédé selon l'une quelconque des revendications précédentes, **caractérisé en ce que** la réaction de réduction est effectuée par le borohydrure de sodium ou de potassium.

10. Procédé selon l'une quelconque des revendications précédentes, **caractérisé en ce que** le composé de formule V formé à l'issu de la réaction de réduction n'est pas isolé du milieu réactionnel et y est directement transformé par addition d'une quantité suffisante d'un aldéhyde R'CHO pour conduire à un composé de formule VII 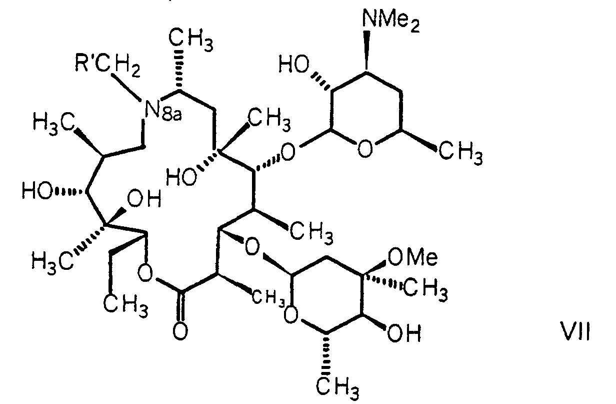 dans laquelle R' représente un atome d'hydrogène, un groupement alkyle en C₁-C₉, alkényle en C₂-C₉.

11. Procédé selon la revendication 10, **caractérisé en ce que** l'aldéhyde utilisé est un aldéhyde en C₁ à C₄.

12. Procédé selon la revendication 10 ou 11, **caractérisé en ce qu'**on utilise à titre d'aldéhyde, du formol pour conduire à la 9-déoxo-8a-aza-8a-méthyl-8a-homoérythromycine A de formule Vl suivante :

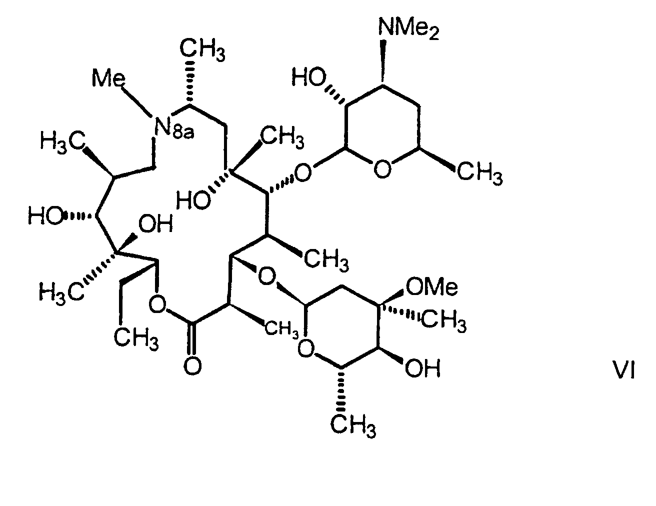

## Patentansprüche

1. Verfahren zur Herstellung von 9-Deoxy-8a-aza-8a-homoerythromycin A der Formel V 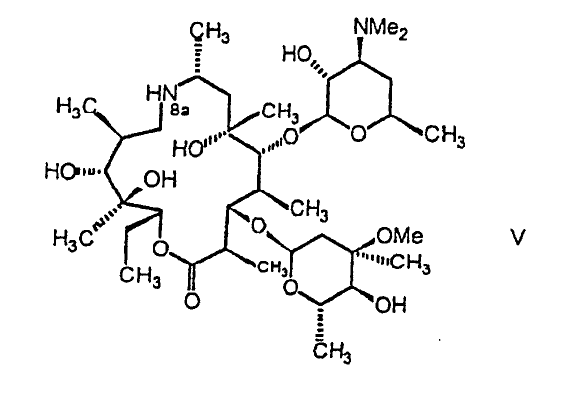 durch stereospezifische Beckmann-Umlagerung in einem Reaktionsmedium unter Verwendung von Pyridin als Hauptlösungsmittel einer Verbindung der Formel II 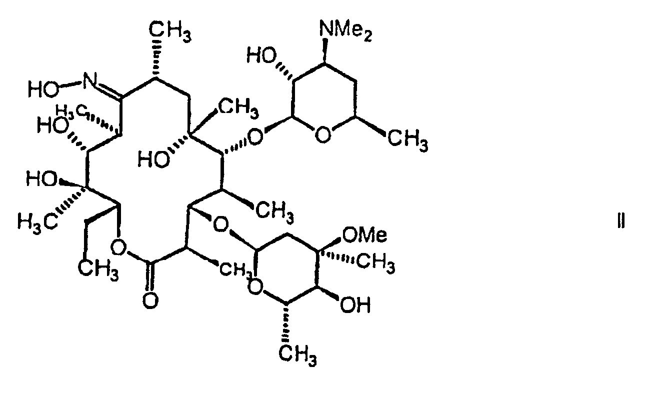 über zwei intermediäre Imidate III und IV 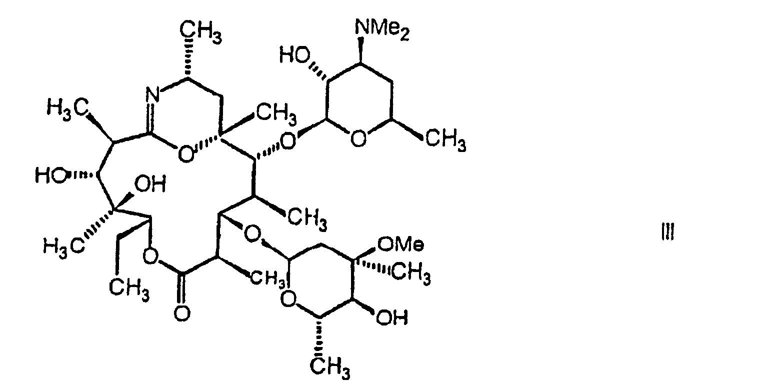 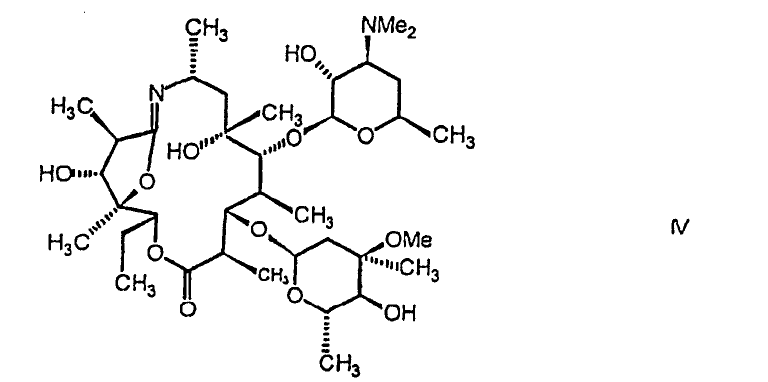 und anschließende Reduktion der Verbindungen III und IV, **dadurch gekennzeichnet, dass** die in dem Reaktionsmedium der Beckmann-Umlagerung gebildeten Verbindungen III und IV nicht aus dem Medium isoliert werden und nach Extraktion aus dem Pyridin mit einem mit diesem mischbaren Kohlenwasserstoff, in dem die Imidate III und IV in Salzform unlöslich sind, direkt in dem Reduktionsschritt mit einer ausreichenden Menge Borhydrid umgesetzt werden.

2. Verfahren nach Anspruch 1, **dadurch gekennzeichnet, dass** die Beckmann-Umlagerung mit Sulfonylchlorid, vorzugsweise ausgewählt aus Tosylchlorid, Benzolsulfonylchlorid und Mesylchlorid, durchgeführt wird.

3. Verfahren nach Anspruch 1 oder 2, **dadurch gekennzeichnet, dass** der Kohlenwasserstoff aus linearen, verzweigten oder cyclischen Kohlenwasserstoffen mit 5 bis 15 Kohlenstoffatomen, insbesondere aus Pentan, Cyclohexan, Methylcyclohexan und Heptan ausgewählt wird, und vorzugsweise aus Heptan besteht.

4. Verfahren nach einem der vorhergehenden Ansprüche, **dadurch gekennzeichnet, dass** die organischen Lösungsmittel, die in dem Medium für die Beckmann-Umlagerung, das das Pyridin umfasst, vorhanden sind, durch Dekantieren entfernt werden.

5. Verfahren nach einem der Ansprüche 1 bis 4, **dadurch gekennzeichnet, dass** nach der Entfernung der organischen Lösungsmittel von dem Pyridin des Mediums der Beckmann-Umlagerung dem Rückstand das Lösungsmittel der Reduktionsreaktion zugesetzt wird, das Wasser ist.

6. Verfahren nach einem der Ansprüche 1 bis 4, **dadurch gekennzeichnet, dass** nach der Entfernung der organischen Lösungsmittel von dem Pyridin des Mediums der Beckmann-Umlagerung dem Rückstand das Lösungsmittel der Reduktionsreaktion zugesetzt wird, das ein organisches Lösungsmittel, ausgewählt aus C₁-C₁₀-Alkohlen, vorzugsweise Methanol oder Isopropanol, oder ein Gemisch eines vorhergehenden Lösungsmittels mit Wasser ist.

7. Verfahren nach einem der Ansprüche 1 bis 4, **dadurch gekennzeichnet, dass** nach der Entfernung der organischen Lösungsmittel von dem Pyridin des Mediums der Beckmann-Umlagerung dem Rückstand das Lösungsmittel der Reduktionsreaktion zugesetzt wird, das vom Amid-Typ ist und das vorzugsweise aus N,N-Dimethylformamid oder N,N-Dimethylacetamid besteht oder das ein Gemisch eines vorhergehenden Lösungsmittels mit Wasser ist.

8. Verfahren nach einem der Ansprüche 1 bis 4, **dadurch gekennzeichnet, dass** nach der Entfernung der organischen Lösungsmittel von dem Pyridin des Mediums der Beckmann-Umlagerung dem Rückstand das Lösungsmittel der Reduktionsreaktion zugesetzt wird, das vom Typ eines cyclischen Harnstoffs, vorzugsweise 1,3-Dimethyl-3,4,5,6-tetrahydro-2(1H)-pyrimidon (DMPU) oder 1,3-Dimethyl-2-imidazolinon (DMEU), oder ein Gemisch eines vorhergehenden Lösungsmittels mit Wasser ist.

9. Verfahren nach einem der vorhergehenden Ansprüche, **dadurch gekennzeichnet, dass** die Reduktionsreaktion mit Natrium- oder Kaliumborhydrid durchgeführt wird.

10. Verfahren nach einem der vorhergehenden Ansprüche, **dadurch gekennzeichnet, dass** die durch die Reduktionsreaktion gebildete Verbindung V nicht aus dem Reaktionsmedium isoliert wird und dort durch Zugabe einer ausreichenden Menge eines Aldehyds R'CHO direkt umgewandelt wird um zu einer Verbindung der Formel VII zu führen 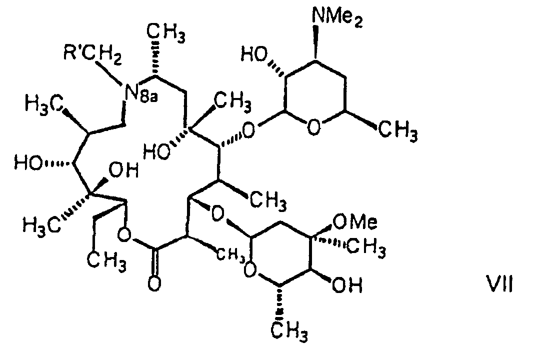 in der R' ein Wasserstoffatom, eine C₁-C₉-Alkylgruppe oder eine C₂-C₉-Alkenylgruppe darstellt.

11. Verfahren nach Anspruch 10, **dadurch gekennzeichnet, dass** der verwendete Aldehyd ein C₁-C₄-Aldehyd ist.

12. Verfahren nach Anspruch 10 oder 11, **dadurch gekennzeichnet, dass** als Aldehyd Formaldehyd verwendet wird, um zu dem 9-Deoxy-8a-aza-8a-methyl-8ahomoerythromycin A der folgenden Formel VI zu führen:

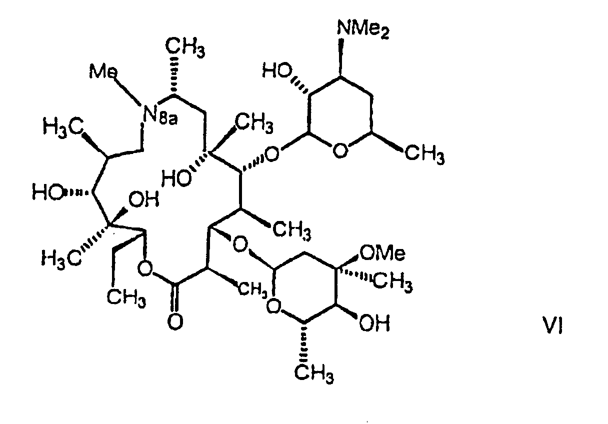

## Claims

1. A process for preparing 9-deoxo-8a-aza-8a-homoerythromycin A with formula V: 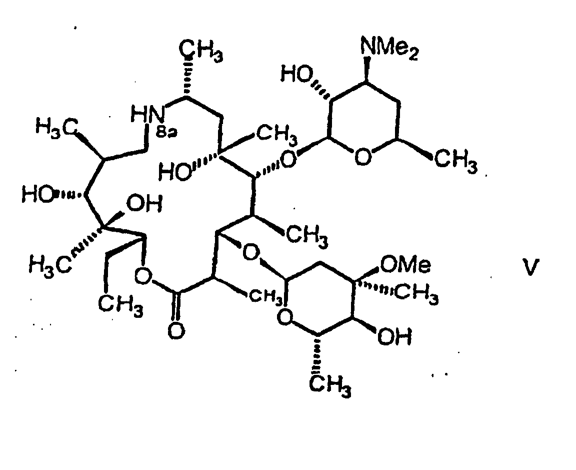 by means of a stereospecific Beckmann rearrangement of a compound with formula II, in a reaction medium using pyridine as the principal solvent: 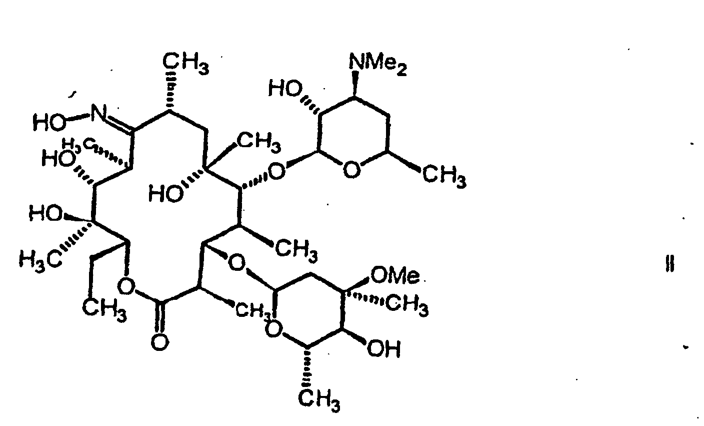 into two imidate intermediates III and IV: 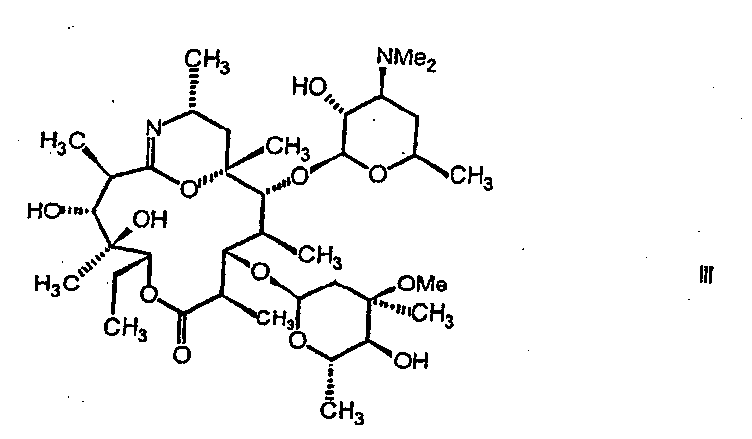 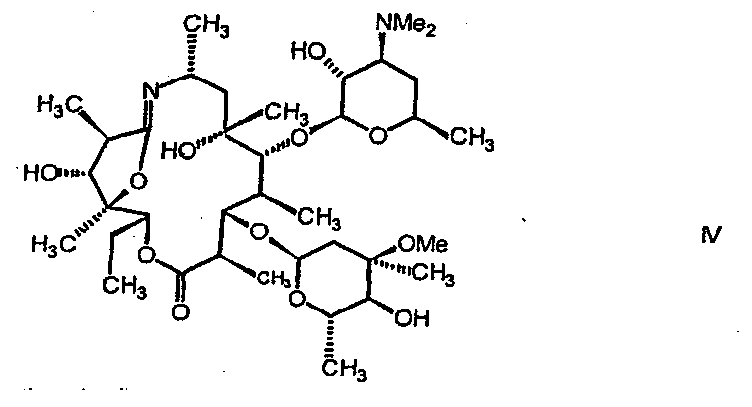 then reacting said compounds III and IV, **characterized in that** said compounds III and IV formed in the Beckmann rearrangement reaction medium are not isolated from said medium and are directly engaged in a reduction step using a sufficient quantity of a borohydride, after extracting pyridine using a hydrocarbon that is miscible therewith and in which the salt form of said imidates III and IV are insoluble.

2. A process according to claim 1, **characterized in that** the Beckmann rearrangement is carried out using a sulphonyl chloride, preferably selected from tosyl chloride, benzenesulphonyl chloride and mesyl chloride.

3. A process according to claim 1 or claim 2, **characterized in that** the hydrocarbon is selected from linear, branched or cyclic hydrocarbons containing 5 to 15 carbon atoms, in particular selected from pentane, cyclohexane, methylcyclohexane or heptane, and preferably consists of heptane.

4. A process according to any one of the preceding claims, **characterized in that** the organic solvents comprising pyridine present in the Beckmann rearrangement medium are eliminated by decanting.

5. A process according to any one of claims 1 to 4, **characterized in that** after eliminating the organic solvents including pyridine from the Beckmann rearrangement medium, the solvent from the reduction reaction, namely water, is added to the residue.

6. A process according to any one of claims 1 to 4, **characterized in that** after eliminating the organic solvents including pyridine from the Beckmann rearrangement medium, the solvent from the reduction reaction, namely an organic solvent selected from C₁-C₁₀ alcohols, preferably methanol or isopropanol, or a mixture of a said solvent and water, is added to the residue.

7. A process according to any one of claims 1 to 4, **characterized in that** after eliminating the organic solvents including pyridine from the Beckmann rearrangement medium, the solvent from the reduction reaction, namely of the amide type and which preferably consists of N,N-dimethylformamide or N,N-dimethylacetamide, or a mixture of a said solvent and water, is added to the residue.

8. A process according to any one of claims 1 to 4, **characterized in that** after eliminating the organic solvents including pyridine from the Beckmann rearrangement medium, the solvent from the reduction reaction, namely of the cyclic urea type, preferably 1,3-dimethyl-3,4,5,6-tetrahydro-2(1H)-pyrimidone (DMPU) or 1,3-dimethyl-2-imidazolinone (DMEU), or a mixture of a said solvent and water, is added to the residue.

9. A process according to any one of the preceding claims, **characterized in that** the reduction reaction is carried out using sodium or potassium borohydride.

10. A process according to any one of the preceding claims, **characterized in that** the compound with formula V formed at the end of the reduction reaction is not isolated from the reaction medium and is directly transformed therein by adding a sufficient quantity of an aldehyde R'CHO to produce a compound with formula VII: 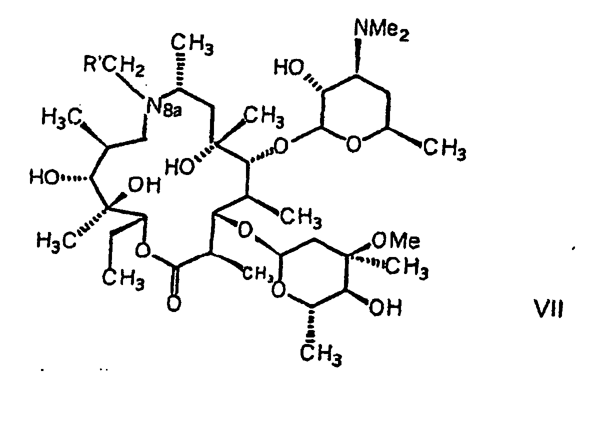 in which R' represents a hydrogen atom, a C₁-C₉ alkyl group or a C₂-C₉ alkenyl group.

11. A process according to claim 10, **characterized in that** the aldehyde used is a C₁ to C₄ aldehyde.

12. A process according to claim 10 or claim 11, **characterized in that** the aldehyde employed is formol, to produce 9-deoxo-8a-aza-8a-methyl-8a-homoerythromycin A with the following formula VI:
